# EUROPEAN PATENT SPECIFICATION

(11) **EP 3 351 620 B1**
(45) Date of publication and mention of the grant of the patent: **02.09.2020**
(21) Application number: 18161278.9
(22) Date of filing: 10.10.2014
(51) Int. Cl.: C12N 5/02, C12N 5/16

(54) **METABOLICALLY OPTIMIZED CELL CULTURE**
METABOLISCH OPTIMIERTE ZELLKULTUR
CULTURE CELLULAIRE À MÉTABOLISME OPTIMISÉ

(30) Priority: 11.10.2013 US 201361889815 P
(43) Date of publication of application: 25.07.2018
(62) Divisional of application: 14851841.8
(73) Proprietor: Regeneron Pharmaceuticals, Inc., Tarrytown, NY 10591-6706 (US)
(72) Inventor: LAWRENCE, Shawn, New York, NY 10989 (US); KIM, Ann, New York, NY 10605 (US); JOHNSON, Amy, New York, NY 10510 (US)
(74) Representative: Adams, Harvey Vaughan John

(56) References cited:
- US-B2- 8 470 552
- BHANU CHANDRA MULUKUTLA ET AL: "On metabolic shift to lactate consumption in fed-batch culture of mammalian cells", METABOLIC ENGINEERING, ACADEMIC PRESS, US, vol. 14, no. 2, 16 December 2011 (2011-12-16), pages 138-149, XP028466094, ISSN: 1096-7176, DOI: 10.1016/J.YMBEN.2011.12.006 [retrieved on 2012-01-08]
- FRANCESCA ZAGARI ET AL: "Lactate metabolism shift in CHO cell culture: the role of mitochondrial oxidative activity", NEW BIOTECHNOLOGY, vol. 30, no. 2, 1 January 2013 (2013-01-01), pages 238-245, XP055295213, NL ISSN: 1871-6784, DOI: 10.1016/j.nbt.2012.05.021
- JAMEY D YOUNG: "Metabolic flux rewiring in mammalian cell cultures", CURRENT OPINION IN BIOTECHNOLOGY., vol. 24, no. 6, 28 May 2013 (2013-05-28), pages 1108-1115, XP055295214, GB ISSN: 0958-1669, DOI: 10.1016/j.copbio.2013.04.016
- ZHOU W ET AL: "Fed-batch culture of recombinant NS0 myeloma cells with high monoclonal antibody production", BIOTECHNOLOGY AND BIOENGINEERING, WILEY & SONS, HOBOKEN, NJ, US, vol. 55, 1 January 1997 (1997-01-01), pages 783-792, XP002170348, ISSN: 0006-3592, DOI: 10.1002/(SICI)1097-0290(19970905)55:5<783: :AID-BIT8>3.0.CO;2-7
- FENG LI ET AL: "Cell culture processes for monoclonal antibody production", MABS, vol. 2, no. 5, 1 September 2010 (2010-09-01), pages 466-479, XP055166177, ISSN: 1942-0862, DOI: 10.4161/mabs.2.5.12720
- HUONG LE ET AL: "Multivariate analysis of cell culture bioprocess data-Lactate consumption as process indicator", JOURNAL OF BIOTECHNOLOGY, vol. 162, no. 2-3, 1 December 2012 (2012-12-01), pages 210-223, XP055287323, AMSTERDAM, NL ISSN: 0168-1656, DOI: 10.1016/j.jbiotec.2012.08.021
- SHEIKHOLESLAMI ZAHRA ET AL: "The impact of the timing of induction on the metabolism and productivity of CHO cells in culture", BIOCHEMICAL ENGINEERING JOURNAL, vol. 79, 8 August 2013 (2013-08-08), pages 162-171, XP028733030, ISSN: 1369-703X, DOI: 10.1016/J.BEJ.2013.07.015
- NINGNING MA ET AL: "A single nutrient feed supports both chemically defined NS0 and CHO fed-batch processes: Improved productivity and lactate metabolism", BIOTECHNOLOGY PROGRESS., vol. 25, no. 5, 27 September 2009 (2009-09-27), pages 1353-1363, XP55287324, US ISSN: 8756-7938, DOI: 10.1002/btpr.238
- NINGNING MA ET AL: "A single nutrient feed supports both chemically defined NS0 and CHO fed-batch processes: Improved productivity and lactate metabolism", BIOTECHNOLOGY PROGRESS., vol. 25, no. 5, 27 September 2009 (2009-09-27), pages 1353-1363, XP055287324, US ISSN: 8756-7938, DOI: 10.1002/btpr.238

## Description

### FIELD OF THE INVENTION

The present invention relates to cells that metabolically shift to lactate consumption in cell culture. A switch to a lactate consumption metabolic profile in seed train culture has beneficial effects on production culture. Upon inoculation of the production reactor, cells exhibit a more efficient lactate metabolism with a low lactate production rate, low peak lactate levels, an early switch to lactate consumption, and subsequently increased productivity in fed-batch mammalian cell culture. Thus, an improved method for large scale production of proteins and/or polypeptides in cell culture is provided.

### BACKGROUND OF THE INVENTION

Biological agents, particularly proteins and polypeptides, are being developed more often as novel pharmaceutical products. Engineered cells that produce unusually high levels of the particular protein of interest have become critically important for successful commercial production of these pharmaceutical interventions. Control and optimization of cell culture conditions varies and has great effect on the level and quality of the therapeutic protein produced in culture.

It is customary to manufacture proteins via cell culture in a batch or fed-batch process. Early stages of inoculum growth after vial thaw include culturing cells in a seed culture. Typically, cells are grown at an exponential growth rate, such as in seed train bioreactors, in order to progressively increase size and/or volume of the cell population. After cell mass is scaled up through several bioreactor stages, cells are then transferred to a production bioreactor while the cells are still in exponential growth (log phase) (Gambhir, A. et al., 2003, J Bioscience Bioeng 95(4):317-327). It is generally considered undesirable to allow cells in batch culture, for example seed culture, to go past the log phase into stationary phase. It has been recommended that cultures should be passaged while they are in log phase, before, cells, e.g. adherent cells, reach confluence due to contact inhibition or accumulation of waste products inhibits cell growth, among other reasons (Cell Culture Basics, Gibco/Invitrogen Online Handbook, www.invitrogen.com; ATCC® Animal Cell Culture Guide, www.atcc.org).

Following transfer to fed-batch culture, cells are cultured for a period of time whereas the composition of the medium is monitored and controlled to allow production of the protein or polypeptide of interest. After a particular yield is reached or cell viability, waste accumulation or nutrient depletion determines that the culture should be terminated, the produced protein or polypeptide is isolated. Many significant advances have been made over the past decade intending to improve recombinant protein yield, which currently reaches titers of multiple grams per liter. Advancements in protein manufacturing processes, as well as in cell line engineering, and cell culture medium and feed development, have contributed to the gain in protein yield.

Fed-batch production involves the addition of small volumes of feed to supplement the nutrients present in the bioreactor as cell growth and product production progresses. It is understood that, in general, mammalian cells tend to continuously metabolize carbohydrates resulting in lactate accumulation, thus requiring base addition to neutralize the lactic acid. Base addition elevates osmolality in the cell medium which in turn greatly restricts the overall achievable cell viability and/or productivity in the bioreactor. Accumulation of lactate in the medium is detrimental to cell growth and is one of the common factors that limit the maximum productivity that can be achieved in batch culture. In a typical batch cell culture, growth and productivity is inhibited after lactate concentration in the culture reaches approximately 30-50 mM and/or ammonia concentration reaches 3-5 mM (Ozturk, S.S., Riley, M.R., and Palsson, B.O. 1992. Biotechnol. and Bioeng. 39: 418-431). To date, widely adopted schemes include nutrient supplementation and the design of chemically defined, serum-free media to support continuous cell growth and optimum product secretion.

Efforts particularly related to reducing the output of metabolic waste products, such as accumulation of lactate, in cell culture have improved the overall quantity of final protein titers. These efforts are focused on controlled glucose or nutrient-limited fed-batch processes (see e.g. WO2004104186; US8192951B2), improved cell culture medium conditions (e.g. US7390660; Zagari, et al., 2013, New Biotechnol., 30(2):238-45), or cellular engineering, including targeting enzymes in the glycolysis pathway (e.g. Kim, S.H. and Lee, G.M., 2007, Appl. Microbiol. Biotechnol. 74, 152-159; Kim, S.H. and Lee, G.M., 2007, Appl. Microbiol. Biotechnol. 76, 659-665; Wlaschin, K.F. and Hu, W-S., 2007, J. Biotechnol. 131,168-176).

Controlled feeding of cells is utilized in an effort to reach a more efficient metabolic phenotype (Europa, A. F., et al., 2000, Biotechnol. Bioeng. 67:25-34; Cruz et al., 1999, Biotechnol Bioeng, 66(2):104-113; Zhou et al., 1997, Cytotechnology 24, 99-108; Xie and Wang, 1994, Biotechnol Bioeng, 43:1174-89). However, this is complicated by the fact that nutrient deprivation as well as rapid changes in, for example, ammonia concentration seen at high cell density fed-batch culture can induce apoptosis ("programmed cell death") (Newland et al., 1994, Biotechnol. Bioeng. 43(5):434-8). Hence, a common optimization approach is to grow cells to moderately high density in fed-batch and then deliberately induce a prolonged, productive stationary phase by, e.g., a temperature or pH change (Quek et al., 2010, Metab Eng 12(2):161-71. doi: 10.1016/j.ymben.2009.09.002. Epub 2009 Oct 13).

Optimization techniques, such as those discussed *supra,* have focused on fed-batch cell culture and this nutrient-dependent process must be adapted for each host cell engineered for production of a polypeptide of interest. Methods to adapt cells to lactate consumers in culture are highly desirous in the process of manufacturing biological therapeutics. Optimizing a cell line with a metabolic phenotype for lactate consumption would prove beneficial to commercial production of polypeptides. Mulukutla B.C. et al. discloses on metabolic shift to lactate consumption in fed-batch culture of mammalian cells. (2011, Metab Eng., 14(2):138-49). Zagari F. et al. discloses lactate metabolism shift in CHO cell culture and the role of mitochondrial oxidative activity (2013, N Biotechnol., 30(2):238-45). Young D.J. discloses metabolic flux rewiring in mammalian cell cultures (2013, Curr Opin Biotechnol., 24(6):1108-1115). Zhou W. et al. discloses fed-batch culture of recombinant NS0 myeloma cells with high monoclonal antibody production (1997, Biotechnol Bioeng., 55(5):783-92). Li F. et al. discloses cell culture processes for monoclonal antibody production (2010, MAbs., 2(5):466-79). Le H. et al. discloses multivariate analysis of cell culture bioprocess data and lactate consumption as process indicator (2012, J Biotechnol. 162(2-3):210-23). US8470552 B2 discloses a strategy to reduce lactic acid production and control PH in animal cell culture. Sheikholeslami Z. et al discloses the impact of the timing of induction on the metabolism and productivity of CHO cells in culture (2013, Biochem Eng J., 79:162-171). Ma N. et al. discloses a single nutrient feed supports both chemically defined NS0 and CHO fed-batch processes: Improved productivity and lactate metabolism (2009, Biotechnol Prog. 25(5):1353-63).

### SUMMARY OF THE INVENTION

The invention provides a method of producing a protein of interest comprising:
(a) culturing cells comprising a nucleic acid sequence encoding said protein of interest in a first cell culture;
(b) determining a metabolic shift to lactate consumption has occurred in said first cell culture;
(c) transferring cells from said first cell culture to a second cell culture after said metabolic shift to lactate consumption has occurred;
(d) maintaining said second cell culture for a period of time so that said protein of interest accumulates in said second cell culture;
(e) harvesting said protein of interest from said second cell culture; and
(f) purifying said protein of interest.

The disclosure provides cells and methods of culturing cells that have metabolically-shifted to lactate consumption. Metabolically adapted cells are ideal for large scale protein production.

One aspect of the invention is a method of culturing cells comprising transferring cells from a first cell culture to a second cell culture after a metabolic shift to lactate consumption in the cells has occurred in the first culture.

Another aspect of the invention provides a method of culturing cells comprising culturing cells in a first cell culture, determining that a metabolic shift to lactate consumption in the cells has occurred in the first cell culture, and transferring the cells to a second cell culture after the metabolic shift to lactate consumption in the cells has occurred, wherein lactate concentration in the second cell culture indicates net lactate consumption during the second culture. In one embodiment, the method further provides a decrease in accumulation of lactate in the second cell culture compared to that determined in an otherwise identical cell culture under otherwise identical conditions except transferring cells to the second cell culture is before a metabolic shift has occurred in the first cell culture.

A second aspect of the invention provides a method of producing a protein comprising transferring cells from a first cell culture to a second cell culture after a metabolic shift to lactate consumption in the cells has occurred, and maintaining the second cell culture for a period of time so that the protein accumulates in the cell culture. In a related aspect, the invention provides a method of producing a protein comprising culturing cells in a first cell culture, determining a metabolic shift to lactate consumption in the cells has occurred in the first cell culture, transferring the cells to a second cell culture after the metabolic shift to lactate consumption in the cells has occurred, and maintaining the second cell culture for a period of time so that the protein accumulates in the cell culture. In one embodiment, the method further provides an increase in productivity in the second cell culture compared to that determined in an otherwise identical cell culture under otherwise identical conditions except transferring cells to the second cell culture is before a metabolic shift has occurred in the first cell culture.

A third aspect of the invention provides an improved method of culturing cells, wherein the cells comprise a gene encoding a polypeptide of interest, comprising the steps of: culturing cells in a first cell culture, maintaining the first cell culture under conditions that allow the expansion of the cell mass, transferring the cells to a second cell culture after the metabolic shift to lactate consumption in the cells has occurred, maintaining the second cell culture under conditions that allow the expression of the polypeptide of interest, and harvesting the polypeptide of interest from the second cell culture. In one embodiment, the method further comprises determining a metabolic shift to lactate consumption in the cells has occurred in the first cell culture.

A fourth aspect of the invention provides an improved method of producing a polypeptide in a cell culture comprising the steps of: transfecting cells with DNA encoding a polypeptide of interest, culturing the cells in a first cell culture, transferring the cells to a second cell culture after the metabolic shift to lactate consumption in the cells has occurred, wherein the polypeptide of interest is expressed under conditions of a second cell culture, and maintaining the second cell culture for a period of time so that the polypeptide accumulates in the cell culture. In one embodiment, the method further comprises determining a metabolic shift to lactate consumption in the cells has occurred in the first cell culture.

A fifth aspect of the invention provides a method of producing a metabolically shifted cell line, comprising the steps of: maintaining a cell population in a first cell culture under conditions that allow the expansion of the cell mass, determining when a metabolic shift to lactate consumption in the cells has occurred, transferring a fraction of the cell population from the first cell culture to a second cell culture after the metabolic shift to lactate consumption in the cells has occurred, maintaining the cell population in the second cell culture for a period of time, and optionally harvesting the cells thus producing the metabolically shifted cell line.

Another aspect of the disclosure provides a metabolically shifted cell line produced by any of the methods of the invention disclosed herein.

In some embodiments, the metabolically shifted cell produced by the method of the invention comprises a nucleic acid sequence stably integrated into the cellular genome wherein the nucleic acid sequence encodes a polypeptide or protein of interest. In other embodiments, the metabolically shifted cell produced by the method of the invention comprises an expression vector encoding a polypeptide or protein of interest.

In one embodiment, the metabolic shift to lactate consumption is detected by pH, lactate or base measurements in the first cell culture. In other embodiments, the cells are transferred to a second cell culture when lactate consumption is detected. In still other embodiments, the metabolic shift to lactate consumption is detected after pH increases in the first cell culture medium without addition of base. In other embodiments, the metabolic shift to lactate consumption is detected when lactate levels plateau in the first cell culture. In still other embodiments, the method further comprises determining the metabolic shift comprising: measuring pH in the first cell culture, adding base to maintain pH above a predetermined lower limit, determining that the pH is above the predetermined lower limit for consecutive intervals, and ceasing the addition of base, thereby determining that the metabolic shift to lactate consumption has occurred in the first cell culture.

In other embodiments, the metabolic shift to lactate consumption is detected by indicators or products of cell metabolism, including but not limited to oxygen consumption, and metabolites such as glycine, tryptophan, phenylalanine, adenine, palmitic acid, glutamic acid, methionine and asparagine. In another embodiment, the metabolic shift to lactate consumption is detected by metabolomic analysis or proteomic analysis.

In one embodiment, the metabolic shift occurs when the cells emerge from log *(i.e.* exponential growth) phase in the first cell culture. In another embodiment, the cells are transferred after the cells emerge from log phase in the first cell culture.

In another embodiment, the metabolic shift occurs when the cells have reached stationary growth phase in the first cell culture. In another embodiment, the cells are transferred after the cells have reached stationary growth phase in the first cell culture.

In one embodiment, the metabolic shift occurs in the first cell culture on or after 3 days of cell growth in the first cell culture. In another embodiment, the metabolic shift occurs in the first cell culture on or after 3.5 days of cell growth in the first cell culture.

In some embodiments, the first cell culture is a seed culture. In some embodiments, the second cell culture is a fed-batch culture. In other embodiments, the second cell culture is a production culture. In other embodiments, the second cell culture is performed in a production bioreactor.

In still other embodiments, the cells are transferred to the second cell culture at a starting cell density of greater than or equal to about 0.5 x 10⁶ cells/mL. In some embodiments, the cells are transferred to the second cell culture at a starting cell density between about 0.5-3.0 x 10⁶ cells/mL.

In some embodiments, lactate concentration in the second cell culture indicates net lactate consumption, for example, net lactate consumption is achieved on or after 2 days, 3 days, 4 days, or 5 days of cell growth in the second cell culture. In more embodiments, the decrease in accumulation of lactate is a reduction in peak lactate concentration in the second cell culture. In other embodiments, the reduction in peak lactate concentration occurs in the second cell culture on or after 5 days of cell growth in the second cell culture. In other embodiments, peak lactate concentration in the second cell culture is less than about 6 g/L, 5 g/L, 4 g/L, 3 g/L, 2 g/L, or less than about 1 g/L.

In some embodiments of the invention, the cell or cells are selected from the group consisting of CHO, COS, retinal, Vero, CV1, HEK293, 293 EBNA, MSR 293, MDCK, HaK, BHK21, HeLa, HepG2, WI38, MRC 5, Colo25, HB 8065, HL-60, Jurkat, Daudi, A431, CV-1, U937, 3T3, L cell, C127 cell, SP2/0, NS-0, MMT, PER.C6, murine lymphoid, and murine hybridoma cells.

### BRIEF DESCRIPTION OF THE DRAWINGS

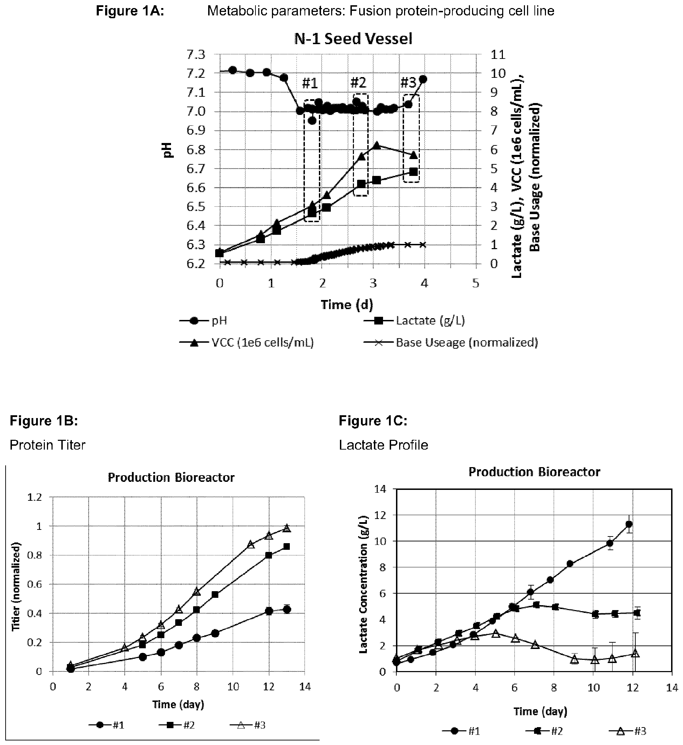

Figure 1: A fusion protein-producing CHO cell line seed vessel was used to inoculate replicate production bioreactors at (**Fig. 1A**) three different metabolic states (online pH and offline lactate) and viable cell counts (VCC). Base usage normalized to 1 for the seed vessel is also shown. The parameters (time, pH, lactate, VCC, and base) for each cell culture (Condition #1, #2, and #3) for which cells were transferred to production bioreactors is indicated by open rectangles (dotted line). All production bioreactors were run with the same operating conditions. The impact of each seed train and its metabolic state on protein titer (**Fig. 1B**) and lactate (**Fig. 1C**) in a production bioreactor is shown. Production bioreactor trendlines represent the average of duplicate bioreactors with error bars that represent ± one standard deviation.

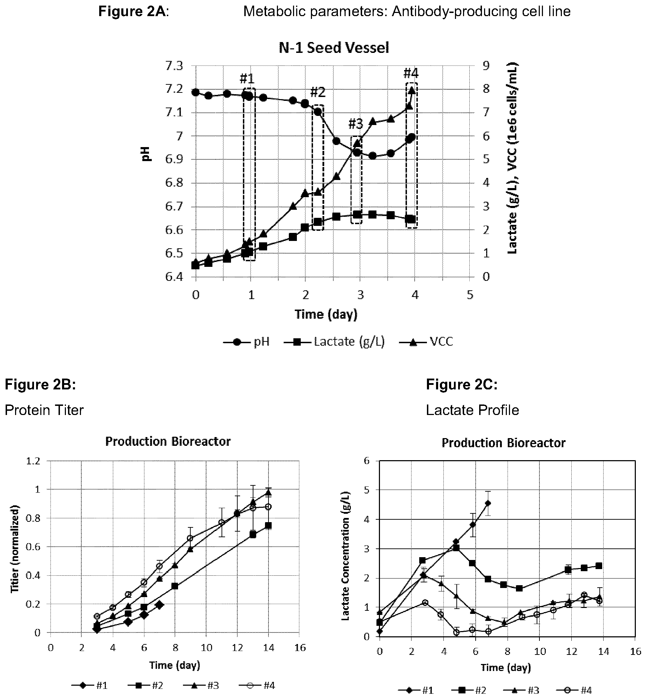

Figure 2: An antibody-producing CHO cell line seed vessel was used to inoculate replicate production bioreactors in a chemically defined process at (**Fig. 2A**) four different metabolic states (offline pH and lactate) and viable cell counts. The parameters (time, pH, lactate, and VCC) for each cell culture (Condition #1, #2, #3 and #4) for which cells were transferred to production bioreactors is indicated by open rectangles (dotted lines). All production bioreactors were run with the same operating conditions. Condition #1 was lost after one week. The impact of each seed train and its metabolic state on a production bioreactor protein titer (**Fig. 2B**) and lactate accumulation (**Fig. 2C**) is also shown. Production bioreactor trendlines represent the average of duplicate bioreactors with error bars that represent ± one standard deviation.

### DETAILED DESCRIPTION OF THE INVENTION

It is to be understood that this invention is not limited to particular methods and experimental conditions described, as such methods and conditions may vary. It is also to be understood that the terminology used herein is for the purpose of describing particular embodiments only, and is not intended to be limiting, since the scope of the present invention is defined by the claims.

As used in this specification and the appended claims, the singular forms "a", "an", and "the" include plural references unless the context clearly dictates otherwise. Thus for example, a reference to "a method" includes one or more methods, and/or steps of the type described herein and/or which will become apparent to those persons skilled in the art upon reading this disclosure.

Unless defined otherwise, all technical and scientific terms used herein have the same meaning as commonly understood by one of ordinary skill in the art to which this invention belongs. Although any methods and materials similar or equivalent to those described herein can be used in the practice of the present invention, particular methods and materials are now described.

### Cell culture

"Batch culture" or "batch mode" as used herein is a phrase that refers to a unit (e.g. culturing vessel) that is filled with cells and with an initial full working volume of medium that is never exchanged. In such a batch culture, all components for cell culturing are supplied to the culturing vessel at the start of the culturing process. The culture usually runs until the nutrients are exhausted or the waste products reach toxic levels and the cells stop growing.

The phrase "seed culture" or "seed train" (also referred to as inoculum train) as used herein includes the inoculation source of a cell population which is allowed to expand in batch culture, or series of batch cultures, until ready for production scale. The seed train expansion process constitutes the initial growth phase of the cells, or inoculum growth phase, following a thaw of frozen cells. The interval between cell thawing and the accumulation of sufficient cell mass to inoculate a production bioreactor constitutes the seed train expansion phase. The cell mass may be scaled up through several bioreactor stages in seed culture, and the cells are grown in cell culture medium under conditions favorable to the survival, growth and viability of the cell culture. It is understood that the seed train is intended to maximize the exponential growth phase, or achieve the maximal growth rate for the particular cell type being cultured. Therefore, passaging of cells from one bioreactor or vessel to another may be one way to achieve maximal growth rate. The precise conditions will vary depending on the cell type, the organism from which the cell was derived, and the nature and character of the expressed polypeptide or protein. A shift to lactate consumption metabolism may occur or be detected in any one of the vessels in a seed train expansion.

The phrase "fed-batch cell culture" or "fed-batch culture" when used herein refers to a batch culture wherein the animal cells and culture medium are supplied to the culturing vessel initially and additional culture nutrients are slowly fed, continuously or in discrete increments, to the culture during culturing, with or without periodic cell and/or product harvest before termination of culture. Fed-batch culture includes "semi-continuous fed-batch culture" wherein periodically whole culture (which may include cells and medium) is removed and replaced by fresh medium. Fed-batch culture is distinguished from simple "batch culture" whereas all components for cell culturing (including the animal cells and all culture nutrients) are supplied to the culturing vessel at the start of the culturing process in batch culture. Fed-batch culture can be further distinguished from perfusion culturing insofar as the supernatant is not removed from the culturing vessel during the process, whereas in perfusion culturing, the cells are restrained in the culture by, e.g., filtration, and the culture medium is continuously or intermittently introduced and removed from the culturing vessel. However, removal of samples for testing purposes during fed-batch cell culture is contemplated. The fed-batch process continues until it is determined that maximum working volume and/or protein production is reached.

The phrase "continuous cell culture" when used herein relates to a technique used to grow cells continually, usually in a particular growth phase. For example, if a constant supply of cells is required, or the production of a particular polypeptide or protein of interest is required, the cell culture may require maintenance in a particular phase of growth. Thus, the conditions must be continually monitored and adjusted accordingly in order to maintain the cells in that particular phase.

The phrase "log phase" as used herein means a period of cell growth typically characterized by cell doubling. The phrases "exponential growth phase" or "exponential phase" are used interchangeably with log phase. In log phase, the number of new cells appearing per unit of time is proportional to the present cell population, hence plotting the natural logarithm of cell number against time produces a straight line. If growth is not limited, doubling will continue at a constant rate so both the number of cells and the rate of population increase doubles with each consecutive time period.

The phrase "stationary phase" as used herein refers to the point where the rate of cell growth equals the rate of cell death. When plotted on a graph, the stationary phase is represented as a plateau, or "smooth," horizontal linear part of the curve.

The term "cell" when used herein includes any cell that is suitable for expressing a recombinant nucleic acid sequence. Cells include those of eukaryotes, such as non-human animal cells, mammalian cells, human cells, or cell fusions such as, for example, hybridomas or quadromas. In certain embodiments, the cell is a human, monkey, ape, hamster, rat or mouse cell. In other embodiments, the cell is selected from the following cells: CHO (e.g. CHO K1, DXB-11 CHO, Veggie-CHO), COS (e.g. COS-7), retinal cells, Vero, CV1, kidney (e.g. HEK293, 293 EBNA, MSR 293, MDCK, HaK, BHK21), HeLa, HepG2, WI38, MRC 5, Colo25, HB 8065, HL-60, Jurkat, Daudi, A431 (epidermal), CV-1, U937, 3T3, L cell, C127 cell, SP2/0, NS-0, MMT cell, tumor cell, and a cell line derived from an aforementioned cell. In some embodiments, the cell comprises one or more viral genes, e.g. a retinal cell that expresses a viral gene (e.g. a PER.C6® cell). In some embodiments, the cell is a CHO cell. In other embodiments, the cell is a CHO K1 cell.

A "cell line" as used herein refers to a cell or cells that are derived from a particular lineage through serial passaging or subculturing of cells. The term "cells" is used interchangeably with "cell population".

Given the current state-of-the-art feeding strategies, CHO cells have achieved cell numbers such as 11 × 10⁶ cells/mL (at day 8) and titers of, for example, 2.3 g/L human IgG (harvested at day 14), numbers that are typical industrial values for CHO cell fed-batch cultures (Kim, BJ, et al. Biotechnol Bioeng. 2012 Jan;109(1):137-45. doi: 10.1002/bit.23289. Epub 2011 Oct 3). Even more than 10 g/L production of antibody has been reported from CHO cells which have been well established as an important industrial mammalian cell line (Omasa et al, Current Pharmaceutical Biotechnology, 2010, 11: 233-240).

The terms "cell culture medium" and "culture medium" refer to a nutrient solution used for growing mammalian cells that typically provides the necessary nutrients to enhance growth of the cells, such as a carbohydrate energy source, essential amino acids, trace elements, vitamins, etc. Cell culture medium may contain extracts, e.g. serum or peptones (hydrolysates), which supply raw materials that support cell growth. Media may contain yeast-derived or soy extracts, instead of animal-derived extracts. Chemically defined medium refers to a cell culture medium in which all of the chemical components are known. Chemically defined medium is entirely free of animal-derived components, such as serum- or animal-derived peptones.

One aspect of the invention relates to a growth phase wherein cell culture conditions are modified to enhance the growth of recombinant eukaryotic cells. In the growth phase, a basal culture medium and cells are supplied to a culturing vessel in batch.

The culturing vessel is inoculated with cells. A suitable seeding density for the initial cell growth phase varies depending on the starting cell line, for example in the range of 0.2 to 3 x 10⁶ cells/mL. Culturing vessels include, but are not limited to well plates, T-flasks, shake flasks, stirred vessels, spinner flasks, hollow fiber, air lift bioreactors, and the like. A suitable cell culturing vessel is a bioreactor. A bioreactor refers to any culturing vessel that is manufactured or engineered to manipulate or control environmental conditions. Such culturing vessels are well known in the art.

Bioreactor processes and systems have been developed to optimize gas exchange, to supply sufficient oxygen to sustain cell growth and productivity, and to remove CO₂. Maintaining the efficiency of gas exchange is an important criterion for ensuring successful scale up of cell culture and protein production. Such systems are well-known to the person having skill in the art.

The exponential growth phase or seed culture *(i.e.* first cell culture) is typically followed by a distinct second culture, known as the polypeptide production phase. In one embodiment, cells undergoing a metabolic shift to lactate consumption in a first cell culture are transferred to a second cell culture. In one embodiment, the second cell culture is carried out in a different culturing vessel from the cell growth phase or seed culture. In some embodiments, the second cell culture takes place in a production bioreactor. In this context, transferring cells refers to the extraction of a fraction of the cell population from the first cell culture vessel and placing the cell population fraction into a second cell culture vessel to initiate the second cell culture.

In other aspects, transferring cells may refer to a volume of cells containing the cells of the first cell culture is placed in a different vessel and the inoculum volume is a fraction of the final volume of the second cell culture, for example about 20%, 30%, 40%, or 50%, or 60%, or 70% or 80% of the final volume. In other aspects, transferring cells may refer to a volume of cells containing the cells of the first cell culture remain in the starting vessel and medium is added so that the initial volume (first cell culture) is a fraction of the final volume of the second cell culture. In this context, the first cell culture is diluted, thereby transferring cells to a second cell culture.

The phrase "emerge from" or "emerges from" as used herein refers to a change from one phase to another phase, or about to change from one phase to another phase. Emerging from a particular phase, for example a growth phase, includes the time period where measurements indicate that a first phase is slowing down or nearly complete, and the subsequent phase is beginning. Emerging from log phase, for example, indicates that cells are ending log phase, and/or are starting or have reached stationary phase. Growth phases are typically measured by viable cell concentration.

The phrase "cell density" refers to the number of cells per volume of sample, for example as number of total (viable and dead) cells per mL. The number of cells may be counted manually or by automation, such as with a flow cytometer. Automated cell counters have been adapted to count the number of viable or dead or both viable/dead cells using for example a standard tryptan blue uptake technique. The phrase "viable cell density" or "viable cell concentration" refers to the number of viable cells per volume of sample (also referred to as "viable cell count"). Any number of well-known manual or automated techniques may be used to determine cell density. Online biomass measurements of the culture may be measured, where the capacitance or optical density is correlated to the number of cells per volume.

Final cell density in a first cell culture, such as seed train density, varies depending on the starting cell line, for example in the range of about 1.0 to 10 x 10⁶ cells/mL. In some embodiments, final seed train density reaches 1.0 to 10 x 10⁶ cells/mL prior to transfer of cells to a second cell culture. In other embodiments, final seed train density reaches 5.0 to 10 x 10⁶ cells/mL prior to transfer of cells to a second cell culture.

In some embodiments, a fraction of the cell population in the first cell culture is transferred to the second cell culture. In other embodiments, the cell population in the first cell culture is transferred to the second cell culture such that the first cell culture is a fraction of the second cell culture. The starting cell density of the second culture may be chosen by the person of ordinary skill in the art. In some embodiments, the starting cell density in the second cell culture is between about 0.5 x 10⁶ cells/mL to about 3.0 x 10⁶ cells/mL. In other embodiments, the starting cell density in the second cell culture is about 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, or 3.0 x 10⁶ cells/mL.

In certain embodiments, the cell supernatant or cell lysate is harvested following the production phase. In other embodiments, the polypeptide or protein of interest is recovered from the culture medium or cell lysate, using techniques well known in the art.

The properties of the cells and the location of the produced product dictate the method used for growth and production, and consequently the selection of a suitable type of bioreactor or culturing vessel. (Bleckwenn, NA and Shiloach, J. 2004 "Large-scale cell culture" Curr Protoc Immunol. 59: Appendix 1U.1-Appendix 1U.44.)

### Metabolic Shift

The phrase "metabolic shift" when used herein refers to a change in cell metabolism, or use of carbon nutrient sources, from lactate production to net lactate consumption. While not being bound to any one theory, the most common carbon nutrient sources in serum-free media are glucose and glutamine, which support rapid cell growth. Glucose may be completely oxidized to CO₂ and H₂O, or, based on the availability of oxygen, be converted to lactate such as in aerobic glycolysis. Fast growing cells consume glucose and glutamine quickly, leading to incomplete oxidative metabolism and, hence, excess lactate production. Carbohydrate metabolism may switch to lactate consumption, and thus reduce the accumulation of lactate.

The phrase "lactate consumption" when used herein refers to the use of lactate as a carbon source in cell metabolism.

The phrase "net lactate consumption" when used herein refers to lactate consumption whereas cells are simultaneously consuming lactate and producing lactate as a byproduct of cell metabolism, and overall rate of consumption is greater than or equal to the rate of production of lactate. When net lactate consumption is increased, overall accumulation of lactate in a cell culture medium is decreased.

Upon initiation of a fed-batch culture, accumulation of lactate, and possibly ammonia, cause the viability of cells to decrease quickly. It has been reported that in fed-batch cultures that did not metabolically shift, none could achieve over 90% viability when the cell concentration had reached its maximum. (Xie and Wang, 1994, Biotechnol. Bioeng. 43(11):1175-1189). Such a metabolic shift, although desirable for optimum process performance, is neither generic nor easily controlled (Zagari, et al., 2013, New Biotechnol. 30(2):238-245). The inventors have discovered that the time and conditions for transfer of cells from a first batch culture (for example, a seed culture) to second batch culture (for example, a fed-batch culture or production culture) has a significant impact on final protein titer. It has been determined unexpectedly that cells cultured for a longer period of time in a first batch culture will switch to lactate consumption and confer a metabolic preference, or metabolic phenotype, for consumption of lactate. It is an objective of this invention to create cells in a constant metabolically shifted state, hence cells with a metabolic memory for lactate consumption. The method of the invention is well-suited for preconditioning cells into a metabolically shifted state such that the cells may be used in any second or subsequent cell culture where lactate consumption is preferred.

In one embodiment, overall accumulation of lactate decreases in the second cell culture. In some embodiments, net lactate consumption is achieved during the second cell culture, for example, net lactate consumption is achieved on or after 2 days, 3 days, 4 days, or 5 days of cell growth in the second cell culture. In more embodiments, the decrease in accumulation of lactate is a reduction in peak lactate concentration in the second cell culture. In other embodiments, the reduction in peak lactate concentration occurs in the second cell culture on or after 5 days of cell growth in the second cell culture. In other embodiments, peak lactate concentration in the second cell culture is less than about 6 g/L, 5 g/L, 4 g/L, 3 g/L, 2 g/L, or less than about 1 g/L.

In some embodiments, metabolically shifted cells produce at least 2-fold, or 3-fold, or 4-fold, or 5-fold, or up to 10-fold lower lactate concentration values in a second cell culture. In some further embodiments, lower lactate concentration values in a second cell culture or overall decreased accumulation of lactate in the second cell culture is compared to that determined in an otherwise identical cell culture under otherwise identical conditions except transferring cells to the second cell culture is before a metabolic shift has occurred in the first cell culture. In still other embodiments, overall accumulation of lactate decreases in the second cell culture on or after 5 days of cell growth in the second cell culture.

In another embodiment, overall product titer increases in the second cell culture. In other embodiments, metabolically shifted cells produce at least 2-fold, or 2.5-fold, 3-fold, or 4-fold, or 5-fold, or up to 10-fold higher product titer in a second cell culture. In still other embodiments, higher protein titer values in a second cell culture is compared to that determined in an otherwise identical cell culture under otherwise identical conditions except transferring cells to the second cell culture is before a metabolic shift has occurred in the first cell culture.

Optimizing metabolic control of cells in culture prior to the fed-batch or production stage has many advantages. Metabolic shift to lactate consumption in a first culture may be determined by multiple parameters. Determining a metabolic shift comprises a number of methods known to the skilled artisan for determining the metabolic state of growing cells.

Measurement of lactate concentration values in a first cell culture may be done by a variety of bioassay systems and kits well known to the person skilled in the art, such as analyzers using electrochemistry (e.g. Bioprofile® Flex, Nova Biomedical, Waltham, MA), or Raman spectroscopy, and may be used for offline or online monitoring of lactate accumulation in cell culture.

It is understood that lactate accumulation has a detrimental effect on cell culture, and subsequently has a negative effect on protein product yield.

In one embodiment, the metabolic shift is determined in a first cell culture when the net accumulation of lactate slows or ceases.

In one embodiment, the metabolic shift to lactate consumption is detected by lactate measurements in the first cell culture. In some embodiments, the metabolic shift is determined in a first cell culture when a plateau, or essentially horizontal line, is determined on a graph representing the measurement of consecutive lactate concentration values in the culture. In other embodiments, the lactate concentration value remains below the upper tolerance limit for consecutive measurements. In still other embodiments, the upper tolerance limit for lactate concentration is no greater than 4 g/L. It is understood that lactate levels plateau when the cells undergo net lactate consumption.

In other embodiments, determining the metabolic shift comprises measuring lactate in the first cell culture at intervals, and determining that the lactate is below the predetermined upper limit for consecutive intervals, thereby determining that the metabolic shift to lactate consumption in the cells has occurred.

pH management and control is an important aspect of maintaining cells in a bioreactor culture. The growth of most cells is optimal within narrow limits of pH. Generally, cell culture is maintained at a neutral pH of 7.0, within a range of upper and lower set-point values. Set point values are determined by the person skilled in the art depending on the particular cell line in culture, the medium composition and the optimal conditions for growth for that cell. As used herein, the expression "neutral pH" means a pH of about 6.85 to about 7.4. The expression "neutral pH" includes pH values of about 6.85, 6.9, 6.95, 7.0, 7.05, 7.1, 7.15, 7.2, 7.25, 7.3, 7.35, and 7.4

On-line, or "real-time", pH monitoring and addition of base may be accomplished by any number of methods well-known to the person skilled in the art. In an on-line system, real-time measurements of biological and chemical parameters in the cell culture by direct connection to an analyzer provide feedback in order to carry out additional actions, for example adding base or adding nutrients to the culture medium. Off-line measurements may also be done whereas periodic sampling and manual operator intervention takes place. Continuous measurement of pH allows cell medium to be monitored and base is added, for example, if acidity reaches a lower set point value outside of tolerance limits. If the pH reaches the set upper tolerance limits *(i.e.* becomes too basic), CO₂ may be added.

On-line monitoring may be done by a variety of methods. Electrodes, such as flow-through electrodes, are commonly used to measure pH, or other parameters such as dissolved O₂ (dO₂) and temperature, in cell culture medium. Such flow-through electrodes plug directly into any standard strip chart recorder for continuous recording or can be interfaced to any standard laboratory pH or millivolt meter. pH may also be measured by means of an optical measurement with the use of a fluorescent sensor spot mounted in the bioreactor.

Any such monitoring system will integrate a tolerance (or dead-band) limit around set point upper and lower values. The dead-band prevents the dosing system from too rapidly switching on and off. During pH control, no dosing or titration will take place if the pH deviation from the set point is within the tolerance limits. If the pH measurement values are larger than the lower tolerance limit (acidic), then a liquid base (e.g. KOH, NaOH, NaHCO₃) or NH₃ gas will be added. If the pH measurement values are above the upper tolerance limit (basic), an acid or CO₂ gas will be added. The pH set-point and control strategy, e.g., dead-band, are linked to multiple parameters such as dissolved CO₂, base consumption for pH control, and therefore, osmolality. (See *e.g.* Li, F., et al., 2010, mAbs 2(5):466-479.)

In one embodiment, the metabolic shift is determined in a first cell culture when addition (i.e. titration) of base stops. Trending of base includes on-line trending wherein an automated monitoring method may be utilized to determine pH and the periodic addition of base. In the present method, the pH set points may vary but the rise in pH off the lower dead-band are indicative of metabolic shift in the first cell culture. Online and manual methods of measuring base trending are known in the art, including methods to monitor the weight of the vessel, or the flow rate of the pump to detect base addition or stoppage of base addition.

In another embodiment, the metabolic shift is determined in a first cell culture when the addition of base is no longer necessary to raise the pH above the lower tolerance limit.

In some embodiments, the metabolic shift is determined in a first culture when the pH value increases without addition of base. In other embodiments, the pH value increases above the lower tolerance limit for consecutive measurements.

In other embodiments, determining the metabolic shift comprises: (a) tuning a pH detection instrument to detect the noise level in the first cell culture, (b) continuously measuring pH in the first cell culture at regular intervals, (c) adding base as necessary to maintain pH above a predetermined lower limit, (d) determining that the pH is above the predetermined lower limit for several consecutive intervals, and (e) ceasing the addition of base, thereby determining that the metabolic shift to lactate consumption in the cells has occurred.

In one embodiment, the lower tolerance limit is a pH of about 6.5, 6.55, 6.6, 6.65, 6.7, 6.75, 6.8, 6.85, 6.9, 6.95, 7.0, 7.05 or about 7.1.

In some embodiments, the metabolic shift to lactate consumption is detected by indicators or products of cell metabolism in the first cell culture. One such indicator of cell metabolism is oxygen consumption (Zagari, et al., 2013, New Biotechnol. 30(2):238-245). An accurate measure of the rate of oxygen depletion in cell culture medium can be used to determine, the presence of viable cells in the culture following inoculation, as well as the rate of growth of the cells in culture (see, e.g., U.S. Patent No. 6,165,741 and U.S. Patent No. 7,575,890). Measurement of oxygen consumption is well-known in the art.

Other indicators of cell metabolism, such as enzymes and metabolites, may be measured by proteomic or metabolomic techniques, such as immunological arrays, nuclear magnetic resonance (NMR) or mass spectometry. Metabolites, such as glycine, tryptophan, phenylalanine, adenine, palmitic acid, glutamic acid, methinonine and asparagine have been correlated with an increase of cellular biomass (See, *e.g.,*Jain, M., et al, Science. 2012 May 25; 336(6084): 1040-1044. doi:10.1126/science.1218595; and De la Luz-Hdez, K., 2012, Metabolomics and Mammalian Cell Culture, Metabolomics, Dr Ute Roessner (Ed.), ISBN: 978-953-51-0046-1, InTech, Available from: http://www.intechopen.com/books/metabolomics/ metabolomics-and-mammalian-cell-cultures). Any number of molecular changes that coincide with or directly lead to metabolic shift in the first cell culture may be utilized to determine that a metabolic shift has occurred.

### Protein Production

The methods of the invention produce a protein or polypeptide of interest in a cell culture. To enable protein production in the methods of the invention, cells are engineered to recombinantly express the polypeptide or protein of interest.

Cells are transferred to a second cell culture, e.g. a production culture, after the metabolic shift to lactate consumption in the cells has occurred, and will be maintained in the second cell culture for a period of time so that the polypeptide or protein accumulates in the cell culture.

As used herein, a "polypeptide" is a single linear polymer chain of amino acids bonded together by peptide bonds between the carboxyl and amino groups of adjacent amino acid residues. The term "protein" may also be used to describe a large polypeptide, such as a seven transmembrane spanning domain protein, with a particular folded or spatial structure. As such, the term "protein" is meant to include quaternary structures, ternary structures and other complex macromolecules composed of at least one polypeptide. If the protein is comprised of more than one polypeptide that physically associate with one another, then the term "protein" as used herein refers to the multiple polypeptides that are physically coupled and function together as the discrete unit. The term "protein" includes polypeptide.

Examples of polypeptides and proteins produced by the methods of the invention include antibodies, fusion proteins, Fc-fusion proteins, receptors, receptor-Fc fusion proteins, and the like.

The term "immunoglobulin" refers to a class of structurally related glycoproteins consisting of two pairs of polypeptide chains, one pair of light (L) chains and one pair of heavy (H) chains, which may all four be inter-connected by disulfide bonds. The structure of immunoglobulins has been well characterized. See for instance Fundamental Immunology Ch. 7 (Paul, W., ed., 2nd ed. Raven Press, N. Y. (1989)). Briefly, each heavy chain typically comprises a heavy chain variable region (abbreviated herein as V_{H} or VH) and a heavy chain constant region (C_{H}). The heavy chain constant region typically comprises three domains, C_{H}1, C_{H}2, and C_{H}3. The C_{H}1 and C_{H}2 domains are linked by a hinge. Each light chain typically comprises a light chain variable region (abbreviated herein as V_{L} or VL) and a light chain constant region. There are two types of light chains in humans, and other mammals: kappa (κ) chain and lambda (λ) chain. The light chain constant region typically comprises one domain (C_{L}). The V_{H} and V_{L} regions may be further subdivided into regions of hypervariability (or hypervariable regions which may be hypervariable in sequence and/or form of structurally defined loops), also termed complementarity determining regions (CDRs), interspersed with regions that are more conserved, termed framework regions (FRs). Each V_{H} and V_{L} is typically composed of three CDRs and four FRs, arranged from amino-terminus (N-terminus) to carboxy-terminus (C-terminus) in the following order: FR1, CDR1, FR2, CDR2, FR3, CDR3, FR4 (see also Chothia and Lesk J. Mol. Biol. 196, 901-917 (1987)). Typically, the numbering of amino acid residues in this region is according to IMGT, Sequences of Proteins of Immunological Interest, 5th Ed. Public Health Service, National Institutes of Health, Bethesda, MD. (1991), or by the EU numbering system of Kabat (also known as "EU numbering" or "EU index"), e.g., as in Kabat, E.A. et al. Sequences of Proteins of Immunological interest. 5th ed. US Department of Health and Human Services, NIH publication No. 91-3242 (1991).

The term "Fc" refers to a portion of a heavy chain constant region that comprises at least the CH2 and CH3 domains that typically bind to an Fc receptor e.g., an FcγR, namely FcγRI (CD64), FcγRII (CD32), FcγRIII (CD16) or an FcRn, *i.e*., a neonatal Fc receptor. It is understood that an Fc-fusion protein may contain all or part of a native Fc domain or contain deletions, substitutions, and/or insertions or other modifications that render it unable to bind any Fc receptor, therefore rendering the domain non-functional or "effectorless" in terms of its typical biological function as achieved through an Fc receptor.

The term "antibody" (Ab) as used herein, refers to an immunoglobulin molecule, or a derivative thereof, which has the ability to specifically bind to an antigen. The variable regions of the heavy and light chains of the immunoglobulin molecule contain a binding domain that interacts with an antigen as outlined above under "immunoglobulin". An antibody may also be a bispecific antibody, diabody, or similar molecule (see for instance Holliger, et al., 1993, PNAS USA 90(14), 6444-8, for a description of diabodies). Further, it has been shown that the antigen-binding function of an antibody may be performed by fragments of a full-length antibody, *i.e.* "antigen-binding fragments" or "antigen-binding proteins". As with full antibody molecules, antigen-binding proteins may be monospecific or multispecific (e.g., bispecific). Examples of binding molecules or fragments encompassed within the term "antibody" include, but are not limited to (i) a Fab' or Fab fragment, a monovalent fragment consisting of the V_{L}, V_{H}, C_{L} and C_{H}1 domains, or a monovalent antibody as described in the international patent publication number WO2007059782; (ii) F(ab')₂ fragments, bivalent fragments comprising two Fab fragments linked by a disulfide bridge at the hinge region; (iii) a Fd fragment consisting essentially of the V_{H} and C_{H}1 domains; (iv) a Fv fragment consisting essentially of a V_{L} and V_{H} domains, (v) a dAb fragment (Ward et al., 1989, Nature 341, 544-546), which consists essentially of a V_{H} domain and also called domain antibodies (Holt et al, 2003, Trends Biotechnol. 21(11):484-90); (vi) camelid or nanobodies (Revets et al., 2005, Expert Opin Biol Ther. 5(1):111-24) and (vii) an isolated complementarity determining region (CDR).

The terms "monoclonal antibody" or "monoclonal antibody composition" as used herein refer to a preparation of antibody molecules of single molecular composition. A monoclonal antibody composition displays a single binding specificity and affinity for a particular epitope.

The term "human antibody", as used herein, is intended to include antibodies having variable and constant regions derived from human germline immunoglobulin sequences. Human antibodies may include amino acid residues not encoded by human germline immunoglobulin sequences (e.g., mutations introduced by random or site- specific mutagenesis *in vitro* or during gene rearrangement or by somatic mutation *in vivo*). The term "mouse or murine monoclonal antibody" refers to antibodies displaying a single binding specificity which have variable and constant regions derived from murine or mouse germline immunoglobulin sequences.

The term "fusion protein" as used herein includes Fc fusion protein and receptor-Fc fusion protein. A fusion protein may be any polypeptide formed by expression of a chimeric gene made by combining more than one DNA sequence of different origins, typically by cloning one gene into an expression vector in frame with a second gene such that the two genes are encoding one continuous polypeptide.

In one aspect, the invention provides a method described herein for producing a recombinant polypeptide or protein of interest. In some embodiments, the recombinant polypeptide or protein of interest is selected from the group consisting of an antibody, antigen-binding protein, fusion protein, Fc fusion protein, and receptor-Fc fusion protein.

### Cell expression systems

The use of cell expression systems is a prerequisite for high production of such polypeptides or proteins in cell culture.

A product according to the invention is a polypeptide, or a protein, which is expressed in the cells and is harvested from the cultivation system, *i.e.* the cells and/or the cell medium. It can be any polypeptide or protein of interest (*supra*).

Expression vectors typically use strong gene promoters to drive product mRNA transcription. In a further aspect, the disclosure relates to an expression vector encoding a polypeptide, e.g. an antibody, antigen-binding protein or fusion protein, of interest. Such expression vectors may be used in the methods of the invention for recombinant production of polypeptides or proteins of interest via cell culture.

An expression vector in the context of the methods of the invention may be any suitable vector, including chromosomal, non-chromosomal, and synthetic nucleic acid vectors (a nucleic acid sequence comprising a suitable set of expression control elements). Examples of such vectors include derivatives of SV40, bacterial plasmids, phage DNA, baculovirus, yeast plasmids, vectors derived from combinations of plasmids and phage DNA, and viral nucleic acid (RNA or DNA) vectors. Such nucleic acid vectors and the usage thereof are well known in the art (see, for instance, US 5,589,466 and US 5,973,972).

A vector comprising a nucleic acid molecule encoding the polypeptide or protein of interest is provided in the host cell, wherein the nucleic acid molecule is operatively linked to an expression control sequence suitable for expression in a mammalian host cell.

Expression control sequences are engineered to control and drive the transcription of polypeptide-encoding genes of interest, and subsequent expression of polypeptides or proteins in various cell systems. Plasmids combine an expressible gene of interest with expression control sequences *(i.e.* expression cassettes) that comprise desirable elements such as, for example, promoters, enhancers, selectable markers, operators, *etc.* In an expression vector nucleic acid molecules may comprise or be associated with any suitable promoter, enhancer, selectable marker, operator, repressor protein, polyA termination sequences and other expression-facilitating elements.

"Promoter" as used herein indicates a DNA sequence sufficient to direct transcription of a DNA sequence to which it is operably linked, i.e., linked in such a way as to control transcription of nucleotide sequence. The expression of a nucleotide sequence may be placed under control of any promoter or enhancer element known in the art. Examples of such elements include strong expression promoters (e. g., human CMV IE promoter/enhancer or CMV major IE (CMV-MIE) promoter, as well as RSV, SV40 late promoter, SL3-3, MMTV, ubiquitin (Ubi), ubiquitin C (UbC), and HIV LTR promoters).

In some embodiments, the vector comprises a promoter selected from the group consisting of SV40, CMV, CMV-IE, CMV-MIE, RSV, SL3-3, MMTV, Ubi, UbC and HIV LTR.

Nucleic acid molecules encoding the polypeptide or protein of interest may also be operatively linked to an effective poly (A) termination sequence, an origin of replication for plasmid product in E. coli, an antibiotic resistance gene as selectable marker, and/or a convenient cloning site (e.g., a polylinker). Nucleic acids may also comprise a regulatable inducible promoter (inducible, repressable, developmentally regulated) as opposed to a constitutive promoter such as CMV IE (the skilled artisan will recognize that such terms are actually descriptors of a degree of gene expression under certain conditions).

Selectable markers are elements well-known in the art. Under the selective conditions, only cells that express the appropriate selectable marker can survive. Commonly, selectable marker genes express proteins, usually enzymes, that confer resistance to various antibiotics in cell culture. In other selective conditions, cells that express a flourescent protein marker are made visible, and are thus selectable. Embodiments include beta-lactamase (bla) (beta- lactam antibiotic resistance or ampicillin resistance gene or ampR), bls (blasticidin resistance acetyl transferase gene), bsd (blasticidin-S deaminase resistance gene), bsr (blasticidin-S resistance gene), Sh ble (Zeocin® resistance gene), hygromycin phosphotransferase (hpt) (hygromycin resistance gene), tetM (tetracycline resistance gene or tetR), neomycin phosphotransferase II (npt) (neomycin resistance gene or neoR), kanR (kanamycin resistance gene), and pac (puromycin resistance gene). Selectable (or selection) markers are typically utilized within stable cell line development.

In certain embodiments, the vector comprises one or more selectable marker genes selected from the group consisting of bla, bls, BSD, bsr, Sh ble, hpt, tetR, tetM, npt, kanR and pac. In other embodiments, the vector comprises one or more selectable marker genes encoding green fluorescent protein (GFP), enhanced green fluorescent protein (eGFP), cyano fluorescent protein (CFP), enhanced cyano fluorescent protein (eCFP), yellow fluorescent protein (YFP), or the like.

For the purposes of this invention, gene expression in eukaryotic cells may be tightly regulated using a strong promoter that is controlled by an operator that is in turn regulated by a regulatory fusion protein (RFP). The RFP consists essentially of a transcription blocking domain, and a ligand-binding domain that regulates its activity. Examples of such expression systems are described in US20090162901A1.

As used herein "operator" indicates a DNA sequence that is introduced in or near a gene of interest in such a way that the gene may be regulated by the binding of the RFP to the operator and, as a result, prevents or allows transcription of the gene of interest. A number of operators in prokaryotic cells and bacteriophage have been well characterized (Neidhardt, ed. Escherichia coli and Salmonella; Cellular and Molecular Biology 2d. Vol 2 ASM Press, Washington D.C. 1996). These include, but are not limited to, the operator region of the *LexA* gene of *E. coli,* which binds the *LexA* peptide, and the lactose and tryptophan operators, which bind the repressor proteins encoded by the *LacI* and *trpR* genes of E. *coli.* These also include the bacteriophage operators from the lambda P_{R} and the phage P22 *ant*/*mnt* genes which bind the repressor proteins encoded by lambda cl and P22 arc. In some embodiments, when the transcription blocking domain of the RFP is a restriction enzyme, such as Notl, the operator is the recognition sequence for that enzyme. One skilled in the art will recognize that the operator must be located adjacent to, or 3' to the promoter such that it is capable of controlling transcription by the promoter. For example, U.S. Pat. No. 5,972,650, specifies that *tetO* sequences be within a specific distance from the TATA box.

In certain embodiments, the operator is selected from the group consisting of tet operator (tetO), Notl recognition sequence, LexA operator, lactose operator, tryptophan operator and Arc operator (AO). In some embodiments, the repressor protein is selected from the group consisting of TetR, LexA, Lacl, TrpR, Arc, LambdaC1 and GAL4. In other embodiments, the transcription blocking domain is derived from a eukaryotic repressor protein, e.g. a repressor domain derived from GAL4.

In an exemplary cell expression system, cells are engineered to express the tetracycline repressor protein (TetR) and a polypeptide of interest is placed under transcriptional control of a promoter whose activity is regulated by TetR. Two tandem TetR operators (tetO) are placed immediately downstream of a CMV-MIE promoter/enhancer in the vector. Transcription of the gene encoding the protein of interest directed by the CMV-MIE promoter in such vector may be blocked by TetR in the absence of tetracycline or some other suitable inducer (e.g. doxycycline). In the presence of an inducer, TetR protein is incapable of binding tetO, hence transcription and thus translation (expression) of the polypeptide of interest occurs. (See, e.g., US Patent No. 7,435,553.

Such cell expression sytems may be used to "turn on" production of the polypeptide of interest during production culture only. Thus, antibiotics, such a tetracycline or other suitable inducers, may be added to the bioreactor to a first cell culture.

Another exemplary cell expression system includes regulatory fusion proteins such as TetR-ER_{LBD}T2 fusion protein, in which the transcription blocking domain of the fusion protein is TetR and the ligand-binding domain is the estrogen receptor ligand-binding domain (ER_{LBD}) with T2 mutations (ER_{LBD}T2; Feil et al., 1997, Biochem. Biophys. Res. Commun. 237:752-757). When tetO sequences were placed downstream and proximal to the strong CMV-MIE promoter, transcription of the nucleotide sequence of interest from the CMV-MIE/*tetO* promoter was blocked in the presence of tamoxifen and unblocked by removal of tamoxifen. In another example, use of the fusion protein Arc2-ER_{LBD}T2, a fusion protein consisting of a single chain dimer consisting of two Arc proteins connected by a 15 amino acid linker and the ER_{LBD}T2 (*supra*), involves an Arc operator (AO), more specifically two tandem arc operators immediately downstream of the CMV-MIE promoter/enhancer. Cell lines may be regulated by Arc2-ER_{LBD}T2, wherein cells expressing the protein of interest are driven by a CMV-MIE/ArcO2 promoter and are inducible with the removal of tamoxifen. (See, e.g., US 20090162901A1.) In some embodiments, the vector comprises a CMV-MIE/TetO or CMV-MIE/AO2 hybrid promoter.

Suitable vectors used in the methods of the invention may also employ Cre-*lox* tools for recombination technology in order to facilitate the replication of a gene of interest. A Cre-/ox strategy requires at least two components: 1) Cre recombinase, an enzyme that catalyzes recombination between two *lox*P sites; and 2) *lox*P sites (e.g. a specific 34-base pair bp sequence consisting of an 8-bp core sequence, where recombination takes place, and two flanking 13-bp inverted repeats) or mutant *lox* sites. (See, e.g. Araki et al., 1995, PNAS 92:160-4; Nagy, A. et al., 2000, Genesis 26:99-109; Araki et al., 2002, Nuc Acids Res 30(19):e103; and US20100291626A1). In another recombination strategy, yeast-derived FLP recombinase may be utilized with the consensus sequence FRT (see also, e.g. Dymecki, S., 1996, PNAS 93(12): 6191-6196).

In another aspect, a gene (*i.e*. a nucleotide sequence encoding a recombinant polypeptide of interest) is inserted within an expression-enhancing sequence of the expression cassette, and is optionally operably linked to a promoter, wherein the promoter-linked gene is flanked 5' by a first recombinase recognition site and 3' by a second recombinase recognition site. Such recombinase recognition sites allow Cre-mediated recombination in the host cell of the expression system. In some instances, a second promoter-linked gene is downstream (3') of the first gene and is flanked 3' by the second recombinase recognition site. In still other instances, a second promoter-linked gene is flanked 5' by the second recombinase site, and flanked 3' by a third recombinase recognition site. In some embodiments, the recombinase recognition sites are selected from a *lox*P site, a lox511 site, a *lox*2272 site, and a FRT site. In other embodiments, the recombinase recognition sites are different. In a further embodiment, the host cell comprises a gene capable of expressing a Cre recombinase.

In another aspect, the vector comprises a first gene encoding a light chain of an antibody or a heavy chain of an antibody of interest, and a second gene encoding a light chain of an antibody or a heavy chain of an antibody of interest.

It is understood that one or more vectors carrying one or more nucleic acid sequences encoding for and expressing the protein of interest may be employed in such an expression system.

Cells of the disclosure may also be engineered to increase product expression via coexpression of proteins such as chaperones, apoptosis inhibitors, protein degradation inhibitors, or other protein which may enhance the expression or stability of the product.

In some aspects, the vector further comprises an X-box-binding-protein 1 (mXBP1) and/or an EDEM2 gene capable of enhancing protein production/protein secretion through control of the expression of genes involved in protein folding in the endoplasmic reticulum (ER). (See, *e.g.* Ron D, and Walter P., 2007, Nat Rev Mol Cell Biol.8:519-529; Olivari et al., 2005, J. Biol. Chem. 280(4): 2424-2428, Vembar and Brodsky, Nat. Rev. Mol. Cell. Biol. 9(12): 944-957, 2008).

The use of transiently transfected cells which produce rapidly significant quantities of the product may also be carried out for the optimization of a cell culture process, however stable transfection is typically utilized for production scales of large volume.

In the context of the present disclosure, the metabolically shifted cell may contain any or all of the elements of a cell expression system as described herein necessary for the efficient recombinant production of a protein of interest.

In an even further aspect, the disclosure relates to a metabolically shifted recombinant eukaryotic host cell which produces a protein of interest. Examples of host cells include mammalian cells, such as CHO, PER.C6, murine lymphoid, and murine hybridoma cell lines (*supra*). For example, in one aspect, the present disclosure provides a metabolically shifted cell comprising a nucleic acid sequence stably integrated into the cellular genome that comprises a sequence encoding for a protein of interest. In another aspect, the present disclosure provides a metabolically shifted cell comprising a non-integrated (*i*.*e*., episomal) nucleic acid sequence, such as a plasmid, cosmid, phagemid, or linear expression element, which comprises a sequence encoding for a protein of interest.

"Harvesting" or "cell harvesting" takes place at the end of a production batch in an upstream process. Cells are separated from medium by a number of methods such as filtration, cell encapsulation, cell adherence to microcarriers, cell sedimentation or centrifugation. Purification of protein takes place in additional steps to isolate the protein product. Polypeptides or proteins may be harvested from either the cells or cell culture media.

Protein purification strategies are well-known in the art. Soluble forms of the polypeptide, such as antibodies, antibody-binding fragments and Fc-containing proteins, may be subjected to commercially available concentration filters, and subsequently affinity purified by well-known methods, such as affinity resins, ion exchange resins, chromatography columns, and the like. Membrane-bound forms of the polypeptide can be purified by preparing a total membrane fraction from the expressing cell and extracting the membranes with a nonionic detergent such as TRITON® X-100 (EMD Biosciences, San Diego, CA, USA). Cytosolic or nuclear proteins may be prepared by lysing the host cells (via mechanical force, sonication, detergent, etc.), removing the cell membrane fraction by centrifugation, and retaining the supernatant.

In a further aspect, the invention relates to a method for producing an antibody, or antigen-binding protein, or fusion protein of interest, said method comprising the steps of a) culturing cells according to the method as described herein above, b) harvesting the cells, and c) purifying the polypeptide or protein, such as antibody, or antigen-binding protein, or fusion protein, from the cells or cell culture media.

The following examples are provided to describe to those of ordinary skill in the art how to make and use methods and compositions of the invention, and are not intended to limit the scope of what the inventors regard as their invention. Efforts have been made to ensure the accuracy with respect to numbers used (e.g. amounts, concentrations, temperature, etc.) but some experimental errors and deviations should be accounted for.

### EXAMPLES

### Example 1- Determining metabolic shift parameters: Fusion protein-producing cell line

CHO cells were transfected with DNA expressing a fusion protein. The fusion protein-producing CHO cell line was incubated in a seed vessel culture, in proprietary media containing soy, and parameters such as online pH, offline lactate and viable cell count, were measured and recorded to determine metabolic state (see #1, #2, or #3 of Figure 1A). Base usage was also monitored and normalized to 1 for this cell line (also see Figure 1A).

Cells under condition #1 and condition #2 were used to inoculate replicate production bioreactors when the pH was controlling at the bottom end of the control range and lactate and VCC were increasing. Cells under condition #3 were inoculated when the pH was starting to increase off the bottom of the control range, *i.e.* base usage had stopped, indicating lactate remetabolization *(i.e.* consumption). Cell growth in condition #3 had entered post-exponential growth phase. All production bioreactors were run with the same operating conditions.

Product titer (see Figure 1B) and lactate profiles (see Figure 1C) were measured in each production bioreactor using known methods to determine the impact of seed train metabolic state #1, #2 or #3. Production bioreactor trendlines represent the average of duplicate bioreactors with error bars that represent ± one standard deviation.

Condition #3 cells had the most significant effect on the productivity and lactate accumulation in the second cell culture, resulting in a greater than 2-fold increase in product titer (compared to Conditions #1 and #2), in the production bioreactor (see Fig. 1B). Condition #3 cells also resulted in decreased lactate concentration following transfer to the second cell culture (compared to Conditions #1 and #2- see Fig. 1C). Condition #3 cells have a lactate profile indicative of net lactate consumption (see Fig. 1C at 8-12 days of cell culture). Cells transferred from the first culture under Condition #1 *(i.e.* prior to a metabolic shift in first culture) do not achieve net lactate consumption in the production bioreactor.

### Example 2- Determining metabolic shift parameters: Antibody-producing cell line

An antibody-producing CHO cell line seed vessel was used to inoculate replicate production bioreactors similar to Example 1, however in chemically defined medium. Four different metabolic states were measured (monitoring offline pH, lactate and viable cell counts- see #1, #2, #3, and #4 of Figure 2A). VCC continued to increase during the duration of the seed vessel incubation when production bioreactors were inoculated.

Condition #1 was inoculated very early in the seed train when the pH was still at the top end of the control range and when the lactate was low but increasing. Condition #2 was inoculated when the pH was starting to decrease and lactate was increasing and approaching peak levels. Condition #3 was inoculated when the pH was near the bottom of the control range and lactate levels had plateaued. Condition #4 was inoculated when the pH was starting to increase off the bottom of the control range and during lactate remetabolization *(i.e.* lactate consumption). All production bioreactors were run with the same operating conditions. Condition #1 was lost after one week.

The impact of seed train metabolic state on production bioreactor titer (Figure 2B) and lactate (Figure 2C) profiles was determined. Production bioreactor trendlines represent the average of duplicate bioreactors with error bars that represent ± one standard deviation.

Condition #3 and #4 cells had the most significant effect on the productivity in the second cell culture. Condition #3 and #4 cells also resulted in reduced lactate concentration in the production bioreactor (compared to Conditions #1 and #2), which is indicative of a metabolic phenotype for lactate consumption (see Fig. 2B and 2C). Similarly to Example 2, cells transferred from first culture under Condition #1 do not achieve net lactate consumption during the production phase. Conditions #2, #3 and #4 achieve net lactate consumption during the production phase, however Condition #4 is most optimal since net lactate consumption occurs earlier than the other conditions, and the peak lactate level is the lowest.

## Claims

1. A method of producing a protein of interest comprising:
(a) culturing cells comprising a nucleic acid sequence encoding said protein of interest in a first cell culture;
(b) determining a metabolic shift to lactate consumption has occurred in said first cell culture;
(c) transferring cells from said first cell culture to a second cell culture after said metabolic shift to lactate consumption has occurred;
(d) maintaining said second cell culture for a period of time so that said protein of interest accumulates in said second cell culture;
(e) harvesting said protein of interest from said second cell culture; and
(f) purifying said protein of interest.

2. The method of claim 1, wherein lactate concentration in the second cell culture indicates net lactate consumption.

3. The method of claim 1 or 2, wherein the cells are selected from the group consisting of CHO, COS, retinal, Vero, CV1, HEK293, 293 EBNA, MSR 293, MDCK, HaK, BHK21, HeLa, HepG2, WI38, MRC 5, Colo25, HB 8065, HL-60, Jurkat, Daudi, A431, CV-1, U937, 3T3, L cell, C127 cell, SP2/0, NS-0, MMT, PER.C6, murine lymphoid, and murine hybridoma cells.

4. The method of claim 3, wherein the cells are CHO cells.

5. The method of any one of claims 1-4, wherein the protein of interest is selected from the group consisting of an antibody, antigen-binding protein, fusion protein, Fc fusion protein, and receptor-Fc fusion protein.

6. The method of any one of claims 1-5, wherein the protein of interest is an antibody or fusion protein.

7. The method of any one of claims 1-6, wherein the protein of interest is produced in said second cell culture at a titer that is greater than the titer produced in an otherwise identical cell culture under otherwise identical conditions except transferring said cells to the second cell culture is before a metabolic shift to lactate consumption has occurred in the first cell culture.

8. The method of claim 7, wherein the protein of interest is produced in said second cell culture at a titer that is at least 2-fold greater than the titer produced in an otherwise identical cell culture under otherwise identical conditions except transferring said cells to the second cell culture is before a metabolic shift to lactate consumption has occurred in the first cell culture.

9. The method of claim 7, wherein the protein of interest is produced in said second cell culture at a titer that is at least 2-fold, or 2.5-fold, 3-fold, or 4-fold, or 5-fold, or up to 10-fold greater than the titer produced in an otherwise identical cell culture under otherwise identical conditions except transferring said cells to the second cell culture is before a metabolic shift to lactate consumption has occurred in the first cell culture.

10. The method of any one of claims 1-9, wherein the metabolic shift to lactate consumption is detected by pH, lactate or base measurements in the first cell culture.

11. The method of any one of claims 1-9, wherein the metabolic shift to lactate consumption is detected after pH increases in the first cell culture medium without addition of base.

12. The method of any one of claims 1-9, wherein the metabolic shift to lactate consumption is detected when lactate levels plateau in the first cell culture.

13. The method of any one of claims 1-9, wherein the step of determining the metabolic shift comprises:
(a) measuring pH in the first cell culture,
(b) adding base to maintain pH above a predetermined lower limit,
(c) determining that the pH is above the predetermined lower limit for consecutive intervals, and
(d) ceasing the addition of base, thereby determining that the metabolic shift to lactate consumption has occurred in the first cell culture.

14. The method of any one of claims 1-13, wherein the metabolic shift occurs in the first cell culture on or after 3 days of cell growth in the first cell culture.

15. The method of any one of claims 1-14, wherein the cells are transferred after the cells emerge from log phase in the first cell culture or after the cells have reached stationary growth phase in the first cell culture.

16. The method of any one of claims 1-15, wherein the cells are transferred to the second cell culture at a starting cell density of greater than or equal to 0.5 x 10⁶ cells/mL, or at a starting cell density between 0.5-3.0 x 10⁶ cells/mL.

17. The method of any one of claims 1-16, wherein the first cell culture is a seed train culture.

18. The method of any one of claims 1-17, wherein the second cell culture is a production culture.

19. The method of any one of claims 1-18, wherein the first cell culture is a seed train culture, the second cell culture is a production culture, and the second cell culture is carried out in a different culturing vessel from the first cell culture.

20. The method of any one of claims 1-19, wherein the second cell culture is performed in a production bioreactor.

21. The method of any one of claims 1-20, wherein the cell population in the first cell culture is transferred to the second cell culture such that the first cell culture is a fraction of the second cell culture.

22. The method of any one of claims 1-21, wherein the nucleic acid sequence is stably integrated into the cellular genome of the cells.

23. The method of any one of claims 1-21, wherein the cells comprise one or more vectors encoding the protein of interest.

24. The method of any one of claims 1-23, wherein the metabolic shift to lactate consumption in the first cell culture comprises a metabolic shift to net lactate consumption.

## Patentansprüche

1. Verfahren zur Herstellung eines Proteins von Interesse, umfassend:
(a)Kultivieren von Zellen, die eine Nukleinsäuresequenz umfassen, die das Protein von Interesse in einer ersten Zellkultur codieren;
(b)Bestimmen, dass eine Stoffwechselverschiebung beim Laktatverbrauch in der ersten Zellkultur aufgetreten ist;
(c)Transferieren von Zellen von der ersten Zellkultur zu einer zweiten Zellkultur, nachdem die Stoffwechselverschiebung beim Laktatverbrauch aufgetreten ist;
(d)Aufrechterhalten der zweiten Zellkultur für eine Zeitdauer, sodass sich das Protein von Interesse in der zweiten Zellkultur akkumuliert;
(e)Ernten des Proteins von Interesse aus der zweiten Zellkultur; und
(f)Aufreinigen des Proteins von Interesse.

2. Verfahren nach Anspruch 1, wobei die Laktatkonzentration in der zweiten Zellkultur einen Netto-Laktatverbrauch angibt.

3. Verfahren nach Anspruch 1 oder 2, wobei die Zellen aus der Gruppe ausgewählt sind, die aus CHO, COS, Retinal, Vero, CV1, HEK293, 293 EBNA, MSR 293, MDCK, HaK, BHK21, HeLa, HepG2, WI38, MRC5, Colo25, HB 8065, HL-60, Jurkat, Daudi, A431, CV-1, U937, 3T3, L-Zelle, C127-Zelle, SP2/0, NS-0, MMT, PER.C6, murinen Lymphoid- und murinen Hybridomazellen besteht.

4. Verfahren nach Anspruch 3, wobei die Zellen CHO-Zellen sind.

5. Verfahren nach einem der Ansprüche 1 bis 4, wobei das Protein von Interesse aus der Gruppe ausgewählt ist, die aus einem Antikörper, antigenbindendem Protein, Fusionsprotein, Fc-Fusionsprotein und Rezeptor-Fc-Fusionsprotein besteht.

6. Verfahren nach einem der Ansprüche 1 bis 5, wobei das Protein von Interesse ein Antikörper- oder Fusionsprotein ist.

7. Verfahren nach einem der Ansprüche 1 bis 6, wobei das Protein von Interesse in der zweiten Zellkultur mit einem Titer hergestellt wird, der größer ist als der Titer, der in einer ansonsten identischen Zellkultur unter ansonsten identischen Bedingungen hergestellt wird, mit Ausnahme des Transferierens der Zellen zu der zweiten Zellkultur, bevor eine Stoffwechselverschiebung beim Laktatverbrauch in der ersten Zellkultur aufgetreten ist.

8. Verfahren nach Anspruch 7, wobei das Protein von Interesse in der zweiten Zellkultur mit einem Titer hergestellt wird, der um das mindestens 2-Fache größer ist als der Titer, der in einer ansonsten identischen Zellkultur unter ansonsten identischen Bedingungen hergestellt wird, mit Ausnahme des Transferierens der Zellen zu der zweiten Zellkultur, bevor eine Stoffwechselverschiebung beim Laktatverbrauch in der ersten Zellkultur aufgetreten ist.

9. Verfahren nach Anspruch 7, wobei das Protein von Interesse in der zweiten Zellkultur mit einem Titer hergestellt wird, der mindestens um das 2-Fache oder 2,5-Fache, 3-Fache oder 4-Fache oder 5-Fache oder bis zu 10-Fache größer ist als der Titer, der in einer ansonsten identischen Zellkultur unter ansonsten identischen Bedingungen hergestellt wird, mit Ausnahme des Transferierens der Zellen zu der zweiten Zellkultur, bevor eine Stoffwechselverschiebung beim Laktatverbrauch in der ersten Zellkultur aufgetreten ist.

10. Verfahren nach einem der Ansprüche 1 bis 9, wobei die Stoffwechselverschiebung beim Laktatverbrauch durch pH-, Laktat- oder Basenmessungen in der ersten Zellkultur erkannt wird.

11. Verfahren nach einem der Ansprüche 1 bis 9, wobei die Stoffwechselverschiebung beim Laktatverbrauch erkannt wird, nachdem sich der pH-Wert im ersten Zellkulturmedium ohne Hinzufügung von Base erhöht.

12. Verfahren nach einem der Ansprüche 1 bis 9, wobei die Stoffwechselverschiebung beim Laktatverbrauch erkannt wird, wenn der Laktatpegel in der ersten Zellkultur ein Plateau erreicht.

13. Verfahren nach einem der Ansprüche 1 bis 9, wobei der Schritt des Bestimmens der Stoffwechselverschiebung umfasst:
(a) Messen des pH-Werts in der ersten Zellkultur,
(b) Hinzufügen von Base zur Aufrechterhaltung des pH-Werts über einer vorbestimmten Untergrenze,
(c) Bestimmen, dass der pH-Wert für aufeinanderfolgende Intervalle über der vorbestimmten Untergrenze liegt, und
(d) Beenden der Zugabe der Base, wodurch bestimmt wird, dass die Stoffwechselverschiebung beim Laktatverbrauch in der ersten Zellkultur aufgetreten ist.

14. Verfahren nach einem der Ansprüche 1 bis 13, wobei die Stoffwechselverschiebung in der ersten Zellkultur bei oder nach 3 Tagen Zellwachstum in der ersten Zellkultur auftritt.

15. Verfahren nach einem der Ansprüche 1 bis 14, wobei die Zellen transferiert werden, nachdem die Zellen aus der logarithmischen Phase in der ersten Zellkultur austreten oder nachdem die Zellen die stationäre Wachstumsphase in der ersten Zellkultur erreicht haben.

16. Verfahren nach einem der Ansprüche 1 bis 15, wobei die Zellen zur zweiten Zellkultur mit einer Ausgangszelldichte von größer oder gleich 0,5 x 10⁶ Zellen/ml oder mit einer Ausgangszellendichte zwischen 0,5 und 3,0 x 10⁶ Zellen/ml transferiert werden.

17. Verfahren nach einem der Ansprüche 1 bis 16, wobei die erste Zellkultur eine Anzuchtschienenkultur ist.

18. Verfahren nach einem der Ansprüche 1 bis 17, wobei die zweite Zellkultur eine Produktionskultur ist.

19. Verfahren nach einem der Ansprüche 1 bis 18, wobei die erste Zellkultur eine Anzuchtschienenkultur ist, die zweite Zellkultur eine Produktionskultur ist und die zweite Zellkultur in einem anderen Kulturgefäß als die erste Zellkultur durchgeführt wird.

20. Verfahren nach einem der Ansprüche 1 bis 19, wobei die zweite Zellkultur in einem Produktionsbioreaktor durchgeführt wird.

21. Verfahren nach einem der Ansprüche 1 bis 20, wobei die Zellpopulation in der ersten Zellkultur zur zweiten Zellkultur transferiert wird, sodass die erste Zellkultur eine Fraktion der zweiten Zellkultur ist.

22. Verfahren nach einem der Ansprüche 1 bis 21, wobei die Nukleinsäuresequenz in das zelluläre Genom der Zellen stabil integriert ist.

23. Verfahren nach einem der Ansprüche 1 bis 21, wobei die Zellen ein oder mehrere Vektoren umfassen, die das Protein von Interesse codieren.

24. Verfahren nach einem der Ansprüche 1 bis 23, wobei die Stoffwechselverschiebung beim Laktatverbrauch in der ersten Zellkultur eine Stoffwechselverschiebung beim Netto-Laktatverbrauch umfasst.

## Revendications

1. Procédé de production d'une protéine d'intérêt comprenant :
(a) la culture de cellules comprenant une séquence d'acide nucléique codant pour ladite protéine d'intérêt dans une première culture cellulaire ;
(b) la détermination d'un changement métabolique vers une consommation de lactate a eu lieu dans ladite première culture cellulaire ;
(c) le transfert des cellules à partir de ladite première culture cellulaire vers une seconde culture cellulaire après que ledit changement métabolique vers une consommation de lactate a eu lieu ;
(d)le maintien de ladite deuxième culture cellulaire pendant une certaine période de temps afin que ladite protéine d'intérêt s'accumule dans ladite seconde culture cellulaire ;
(e) la récolte de ladite protéine d'intérêt à partir de ladite seconde culture cellulaire ; et
(f) la purification de ladite protéine d'intérêt.

2. Procédé selon la revendication 1, dans lequel la concentration de lactate dans la seconde culture cellulaire indique la consommation nette de lactate.

3. Procédé selon la revendication 1 ou 2, dans lequel les cellules sont choisies dans le groupe constitué des cellules CHO, COS, rétiniennes, Vero, CV1, HEK293, 293 EBNA, MSR 293, MDCK, HaK, BHK21, HeLa, HepG2, WI38, MRC5, Colo25, HB 8065, HL-60, Jurkat, Daudi, A431, CV-1, U937, 3T3, cellule L, cellule C127, SP2/0, NS-0, MMT, PER.C6, cellules lymphoïdes murines et hybridomes murins.

4. Procédé selon la revendication 3, dans lequel les cellules sont des cellules CHO.

5. Procédé selon l'une quelconque des revendications 1 à 4, dans lequel la protéine d'intérêt est sélectionnée parmi le groupe constitué par un anticorps, une protéine de liaison à l'antigène, une protéine de fusion, une protéine de fusion Fc et une protéine de fusion récepteur-Fc.

6. Procédé selon l'une quelconque des revendications 1 à 5, dans lequel la protéine d'intérêt est un anticorps ou une protéine de fusion.

7. Procédé selon l'une quelconque des revendications 1 à 6, dans lequel la protéine d'intérêt est produite dans ladite seconde culture cellulaire à un titre qui est supérieur au titre produit dans une culture cellulaire par ailleurs identique dans des conditions par ailleurs identiques, à l'exception du transfert desdites cellules vers la seconde culture cellulaire qui se fait avant qu'un changement métabolique vers une consommation de lactate n'ait eu lieu dans la première culture cellulaire.

8. Procédé selon la revendication 7, dans lequel la protéine d'intérêt est produite dans ladite seconde culture cellulaire à un titre qui est au moins deux fois supérieur au titre produit dans une culture cellulaire par ailleurs identique dans des conditions par ailleurs identiques, à l'exception du transfert desdites cellules vers la seconde culture cellulaire qui se fait avant qu'un changement métabolique vers une consommation de lactate n'ait eu lieu dans la première culture cellulaire.

9. Procédé selon la revendication 7, dans lequel la protéine d'intérêt est produite dans ladite seconde culture cellulaire à un titre au moins 2 fois, ou 2,5 fois, 3 fois, ou 4 fois, ou 5 fois, ou jusqu'à 10 fois supérieur au titre produit dans une culture cellulaire par ailleurs identique dans des conditions par ailleurs identiques, à l'exception du transfert desdites cellules vers la seconde culture cellulaire qui se fait avant qu'un changement métabolique vers une consommation de lactate n'ait eu lieu dans la première culture cellulaire.

10. Procédé selon l'une quelconque des revendications 1 à 9, dans lequel le changement métabolique vers une consommation de lactate est détecté par des mesures de pH, de lactate ou de base dans la première culture cellulaire.

11. Procédé selon l'une des revendications 1 à 9, dans lequel le changement métabolique vers une consommation de lactate est détecté après l'augmentation du pH dans le premier milieu de culture cellulaire sans ajout de base.

12. Procédé selon l'une quelconque des revendications 1 à 9, dans lequel le changement métabolique vers une consommation de lactate est détecté lorsque les niveaux de lactate atteignent un plateau dans la première culture cellulaire.

13. Procédé selon l'une quelconque des revendications 1 à 9, dans lequel l'étape de détermination du changement métabolique comprend :
(a) la mesure du pH dans la première culture cellulaire,
(b) l'ajout d'une base pour maintenir le pH au-dessus d'une limite inférieure prédéterminée,
(c) le fait de déterminer que le pH est supérieur à la limite inférieure prédéterminée pour des intervalles consécutifs, et
(d) l'arrêt de l'ajout de base, déterminant ainsi que le changement métabolique vers une consommation de lactate s'est produit dans la première culture cellulaire.

14. Procédé selon l'une quelconque des revendications 1 à 13, dans lequel le changement métabolique se produit dans la première culture cellulaire au moment de la croissance cellulaire dans la première culture cellulaire ou après 3 jours celle-ci.

15. Procédé selon l'une quelconque des revendications 1 à 14, dans lequel les cellules sont transférées après que les cellules émergent de la phase logarithmique dans la première culture cellulaire ou après que les cellules ont atteint une phase de croissance stationnaire dans la première culture cellulaire.

16. Procédé selon l'une quelconque des revendications 1 à 15, dans lequel les cellules sont transférées vers la seconde culture cellulaire à une densité cellulaire de départ supérieure ou égale à 0,5 x 10⁶ cellules/ml, ou à une densité cellulaire de départ comprise entre 0,5 et 3,0 x 10⁶ cellules/ml.

17. Procédé selon l'une quelconque des revendications 1 à 16, dans lequel la première culture cellulaire est une culture de chaîne de grains.

18. Procédé selon l'une quelconque des revendications 1 à 17, dans lequel la seconde culture cellulaire est une culture de production.

19. Procédé selon l'une quelconque des revendications 1 à 18, dans lequel la première culture cellulaire est une culture de chaîne de grains et la seconde culture cellulaire est une culture de production, et la seconde culture cellulaire est effectuée dans un récipient de culture différent de la première culture cellulaire.

20. Procédé selon l'une quelconque des revendications 1 à 19, dans lequel la seconde culture cellulaire est effectuée dans un bioréacteur de production.

21. Procédé selon l'une quelconque des revendications 1 à 20, dans lequel la population cellulaire dans la première culture cellulaire est transférée vers la seconde culture cellulaire de sorte que la première culture cellulaire est une fraction de la seconde culture cellulaire.

22. Procédé selon l'une quelconque des revendications 1 à 21, dans lequel la séquence d'acide nucléique est intégrée de manière stable dans le génome cellulaire des cellules.

23. Procédé selon l'une quelconque des revendications 1 à 21, dans lequel les cellules comprennent un ou plusieurs vecteurs qui codent la protéine d'intérêt.

24. Procédé selon l'une quelconque des revendications 1 à 23, dans lequel le changement métabolique vers une consommation de lactate dans la première culture cellulaire comprend un changement métabolique vers une consommation nette de lactate.
